# EUROPEAN PATENT SPECIFICATION

(11) **EP 2 514 384 B1**
(45) Date of publication and mention of the grant of the patent: **25.02.2015**
(21) Application number: 10837775.5
(22) Date of filing: 02.11.2010
(51) Int. Cl.: A61B 18/20, A61N 5/067, A61N 5/06, A61B 18/22

(54) **APPARATUS FOR LIPID REMOVAL USING INFRARED OPO LASER**
VORRICHTUNG ZUR FETTENTFERNUNG MITTELS INFRAROT-OPO-LASER
APPAREIL PERMETTANT UNE ÉLIMINATION DES LIPIDES À L'AIDE D'UN LASER D'OSCILLATEUR PARAMÉTRIQUE OPTIQUE À RAYONNEMENT INFRAROUGE

(30) Priority: 16.12.2009 KR 20090125750
(43) Date of publication of application: 24.10.2012
(73) Proprietor: Industry-Academic Cooperation Foundation, Dankook University, Gyeonggi-do 448-701 (KR)
(72) Inventor: RHEE, Bum Ku, Goyang-si Gyeonggi-do 410-708 (KR); RHEE, Chung-Ku, Cheonan-si Chungcheongnam-do 330-180 (KR)
(74) Representative: Mai, Dörr, Besier
(86) International application number: PCT/KR2010/007669
(87) International publication number: WO 2011/074778

(56) References cited:
- KR-A- 20080 006 574
- KR-B1- 100 742 973
- US-A- 6 096 031
- US-A1- 2006 153 254
- US-A1- 2008 188 835
- US-A1- 2008 188 835
- US-B1- 6 605 080
- US-B1- 6 605 080
- "Periodically Poled Lithium Niobate", , 1 January 2009 (2009-01-01), XP055064388, United Kingdom Retrieved from the Internet: URL:http://www.rayscience.com/PPLN/Covesio n_PPLN_Catalogue.pdf [retrieved on 2013-05-28]

## Description

### Technical Field

The present invention relates to an apparatus for removing fat from a living body using an infrared optical parametric oscillator (OPO) laser, and more particularly to an apparatus of removing fat from a living body using an infrared OPO laser, the apparatus being able to remove fat from a living body in a more energy-efficient, rapid and effective manner by irradiating fat in the living body directly with two infrared lasers having wavelengths of about 2,300 nm and about 1,980 nm, which are generated using a pump laser having a wavelength of about 1,064 nm as a light source.

### Background Art

According to the disclosure of U.S. Patent No. 6,605,080 B1 (hereinafter referred to as "Altshuler et al."), as shown in FIG. 1, the absorption coefficients of laser lights having wavelengths of 930 nm, 1230 nm, 1700 nm and 2300 nm are highest in human fatty tissue, and particularly, these absorption coefficients are at least two times higher than the absorption coefficients of laser lights having the above wavelengths in water that constitutes the majority of the human body. When the epidermis is irradiated with the laser lights of the above wavelengths in view of this advantage, fat tissue can be non-invasively heated, and thus destroyed, thereby reducing body weight. However, it was clinically proven that infrared light having a wavelength of 2,300 nm has a high coefficient of absorption not only in fat tissue, but also in the epidermis. Thus, this light cannot remove fat from the body without damaging the epidermis, and can liquefy fat in the body only by invasive methods. Accordingly, Altshuler et al. also suggests a method of effectively removing fat tissue by selectively using high-output infrared light at a wavelength of 1,200 nm or 2,300 nm.

A further laser system for fat removal is disclosed in US2008/0188835 A1.

In addition, Korean Patent Registration No. 798635 (hereinafter referred to as "Lee et al.") suggests a laser apparatus which can selectively irradiate the inside and outside of the skin with a laser beam and, at the same time, can efficiently remove fat using a laser of a wavelength of 930 nm, which has a high coefficient of absorption in fat, among the wavelengths at which a laser diode can oscillate.

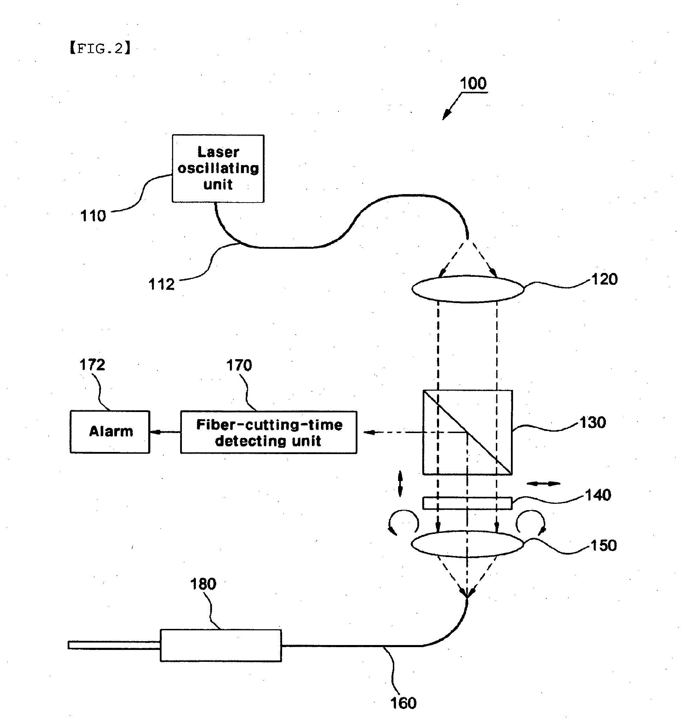

As shown in FIG. 2, the laser apparatus 100 of Lee et al. comprises: a laser oscillating unit 110 for outputting a laser beam; a first optical fiber 112 for guiding the laser beam output from the laser oscillating unit 110; a first parallel light-converting unit 120, which is located at the output terminal of the first optical fiber 112 and serves to convert the laser beam, which is guided through the optical fiber 112, into parallel light so that it is not diffused; a polarizer 130 which serves to linearly polarize the parallel laser beam incident from the first parallel light-converting unit 120 and to prevent the feedback of light, reflected from a second optical fiber 160 and then refracted and phase-delayed, to the laser oscillating unit 110; a phase delay unit 140 which serves to circularly polarize the linearly polarized laser beam incident from the polarizer and to linearly polarize the circularly polarized laser beam reflected from the second optical fiber 160; a convergent lens 150 which converges the laser beam circularly polarized through the phase delay unit 140; a second optical fiber 160 which guides the light converged by the convergent lens 150 to subcutaneous fat; a fiber-cutting-time detecting unit 170 which measures the amount of energy of the laser beam, which is reflected from the terminal of the second optical fiber 160 and is then reflected from the polarizer, and outputs a fiber cutting signal when the measured amount reaches a set amount of energy; and an alarm 172 which outputs an alarm signal based on the fiber cutting signal received from the fiber-cutting-time detecting unit 170.

The apparatus of prior art document 2 may optionally comprise a contact tip 194 or a cannula 180 in the optical fiber 160, so that it can selectively irradiate the inside and outside of the skin with a laser beam. In addition, fat can be efficiently removed using a laser of a wavelength of 930 nm, which has a high coefficient of absorption in fat, among the wavelengths at which a laser diode can oscillate.

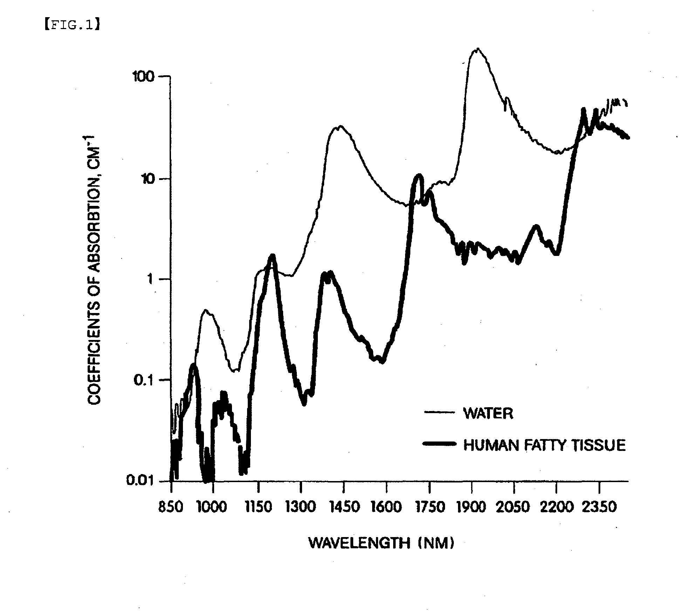

However, as can be seen in FIG. 1, the laser having a 930-nm wavelength laser has an absorption coefficient of only about 0.1, which does not significantly differ from the coefficient of absorption in water. Thus, it has low efficiency in removing fat tissue and has a high risk of damaging other organs.

Accordingly, the present inventors have developed an apparatus of removing fat from a living body using an infrared OPO laser, the apparatus being able to remove fat from a living body using laser lights having specific wavelengths in a more energy-efficient, rapid and effective manner at a very low risk of damaging other organs.

### Disclosure

### Technical Problem

Accordingly, the present invention has been made keeping in mind the above-described problems occurring in the prior art, and an object thereof is to provide an apparatus for removing fat from a living body using an infrared optical parametric oscillator (OPO) laser, the apparatus being able to remove fat from a living body using laser lights having specific wavelengths in a more energy-efficient, rapid and effective manner at a very low risk of damaging other organs.

Another object of the present invention is to provide an apparatus for effectively removing fat by selectively controlling exposure of infrared OPO laser light depending on the size and thickness of the fatty tissue to be removed.

### Technical Solution

In order to accomplish the above objects, in one aspect, the present invention relates to an apparatus for lipid removal using infrared OPO laser, the apparatus comprising: a laser (20) which includes a pump light source; an optical fiber (50) configured to guide the light from the laser (20) to fat tissue; and a needle (60) into which one end of the optical fiber (50) is inserted, wherein the laser (20) is an OPO laser (20) configured to receive the pump light having a wavelength ranging from 1,054 nm to 1,074 nm and output a first laser light having a wavelength ranging from 1,970 nm to 1,990 nm and a second laser light having a wavelength ranging from 2,290 nm to 2,310 nm simultaneously, wherein the apparatus further comprises a dichroic optical filter (30) detachably located in a line along which the light output from the OPO laser (20) progresses, and configured to reflect the first laser light and selectively transmit the second laser light, among the light output from the OPO laser (20).

Preferably, the apparatus further comprises: a convergent lens (40) which converges the laser light's output from the OPO laser (20); an optical fiber (50) which is able to guide the laser lights converged by the convergent lens (40); and a temperature control unit (70) which displays, measures and monitors the temperature of the skin in an area into which the needle (60) is inserted and from which fat is being removed, and cools the skin in the area or stops laser irradiation when the measured temperature is higher than a preset safe temperature.

Preferably, the apparatus further comprises a convergent lens (40) which converges the light passed through the dichroic optical filter (30) from the OPO laser (20).

Preferably, the pump light may be light from a diode-pumped solid-state laser.

Preferably, the pump light may be light from a fiber laser.

Preferably, the infrared OPO laser (20) comprises: an input mirror unit (21) which receives the pump light and reflects infrared light generated in the OPO laser (20); a nonlinear crystal (22) which generates and amplifies the light generated in the OPO laser (20) by interaction with the pump light received from the input mirror unit (21); and an output mirror unit (23) which outputs a portion of the infrared light generated from the nonlinear crystal (22) and reflects the remainder.

Preferably, the nonlinear crystal (22) is a periodically poled ferroelectric crystal which is able to receive the pump light having a wavelength of 1,064 nm and output the first laser light having a wavelength of 1,980 nm and the second laser light having a wavelength of 2,300 nm.

Preferably, the nonlinear crystal (22) is a periodically poled lithium niobate (PPLN; LiNbO3) which is able to receive the pump light having a wavelength of 1,064 nm and output a first laser light having a wavelength of 1,980 nm and the second laser light having a wavelength of 2,300 nm.

Preferably, the nonlinear crystal (22) is a periodically poled MgO-doped stoichiometric lithium tantalate (LiTaO3) which is able to receive the pump light having a wavelength of 1,064 nm and output a first laser light having a wavelength of 1,980 nm and the second laser light having a wavelength of 2,300 nm.

Preferably, the needle (60) comprises a metal needle (61) for skin insertion having a handle (62) formed at one end and a plastic needle (63) which surrounds the metal needle (61) and has an optical fiber (50) fixing protrusion formed at one end and a slant formed at the other end. Alternatively, a metal needle may also be used alone, without the plastic-metal needle.

Preferably, the temperature control unit (70) comprises: a camera (71) for taking an image of the skin in an area from which fat tissue is being removed (fat removal area); a temperature sensor (72) which measures temperature by receiving infrared light emitted from the skin of the fat removal area; a cooler (73) for cooling the skin of the fat removal area; and a processor (74) connected with the camera (71), the temperature sensor (72) and the cooler (73) and in which a color table for expressing the temperature as a color and a safe temperature are input, so that the processor (74) converts the measured temperature, received from the temperature sensor (72) into the color, and controls the cooler (73) to reduce the skin temperature of the fat removal area, or stops laser irradiation when the temperature measured is higher than the safe temperature.

Preferably, the temperature control unit (70) further comprises a display (75) connected to the processor (74) and configured to display a color corresponding to the temperature measured on the image taken by the camera (71).

### Advantageous Effects

According to the present invention, both infrared light having wavelengths both of about 1,980nm and infrared light having a wavelength of about 2,300 nm, which are simultaneously generated in an infrared OPO laser, can be used for fat removal with very high efficiency.

Furthermore, according to the present invention, among the light output from the infrared OPO laser, infrared light having a wavelength of about 1,980 nm has a very high coefficient of absorption in water, and infrared light having a wavelength of about 2,300 nm has a very high coefficient of absorption in fat. Thus, in the case of large or thick fat tissue containing a large amount of water, fat together with water can be removed from the fat tissue, so that the fat can be effectively destroyed in a short time compared to when the fat is destroyed using only infrared light having a wavelength of about 2,300 nm.

In addition, according to the present invention, even in the case in which a fat layer is thin, fat can also be removed even with infrared light having a wavelength of about 2,300 nm, optionally using a dichroic filter. Thus, the surgical system does not need to be altered depending on changes in the thickness of fat tissue that occur during fat removal operation. Moreover, because infrared light having a wavelength of 2,300 nm has a very high coefficient of absorption in fat, it penetrates the skin to a relatively shallow depth, and does not damage the surrounding tissue. Thus, the use of this infrared light enables only fat to be safely removed from an area near the skin or muscle.

### Description of Drawings

FIG. 1 shows the absorption coefficients of laser light in fat and water according to prior art document 1;
FIG. 2 schematically shows the configuration of the apparatus disclosed in prior art document 2;
FIG. 3 shows the specific configuration of one embodiment using an apparatus for removing fat using an infrared OPO according to the present invention;
FIGS. 4 and 5 illustrate the configuration of an apparatus for removing fat using an infrared OPO according to one embodiment of the present invention;
FIG. 6 illustrates the configuration of a plastic-metal needle according to one embodiment of the present invention;
FIGS. 7 and 8 illustrate the configuration of a temperature control unit according to the present invention;
FIG. 9 explains a method of removing fat using an apparatus for removing fat using an OPO laser according to the present invention; and
FIGS. 10 to 12 are flow charts illustrating a method for lipid removal using infrared OPO laser.

### <Description of the Reference Numerals in the Drawings>

10: pump light output laser;
20: OPO laser;
21: input mirror unit;
22: nonlinear crystal;
23: output mirror unit;
30: dichroic optical filter;
40: convergent lens;
50: optical fiber;
60: plastic-metal needle or metal needle
61: metal needle;
62: handle;
63: plastic needle;
64: fixing protrusion;
65: slant;
70: temperature control unit;
71: camera;
72: temperature sensor;
73: cooler;
74: processor;
75: display;
901: skin layer;
902: muscle layer;
903: fat layer;
S10: insertion hole-forming step;
S20: inserting step;
S30: fat liquefying step;
S31: output light control step;
S40: temperature control step;
S41: measurement step;
S42: conversion step;
S43: comparison step;
S44: control step; and
S50: liquefied-fat removing step.

### Best Mode

Hereinafter, preferred embodiments of an apparatus of removing fat using an infrared OPO laser according to the present invention will be described with reference to the accompanying drawings. Further, the size of lines or the size of components, shown in the drawings, may be exaggerated for the clarity and convenience of description. Further, the following terms, which are defined in consideration of functions of the present invention, may be altered depending on users' or operators' intentions or practices. Therefore, the meaning of each term should be interpreted based on the entire disclosure of the specification.

As used herein, the terms "around", "about" or "approximately" shall generally mean within 5%, preferably within 3%, and more preferably within 1% of a given value or range. Numerical quantities given herein are approximate, meaning that the terms "around", "about" or "approximately" can be assumed unless specifically stated.

### Embodiments

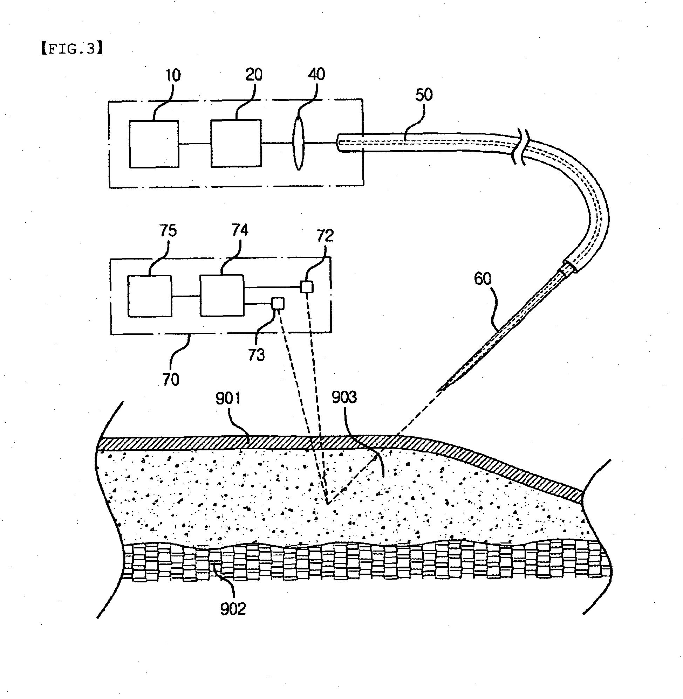

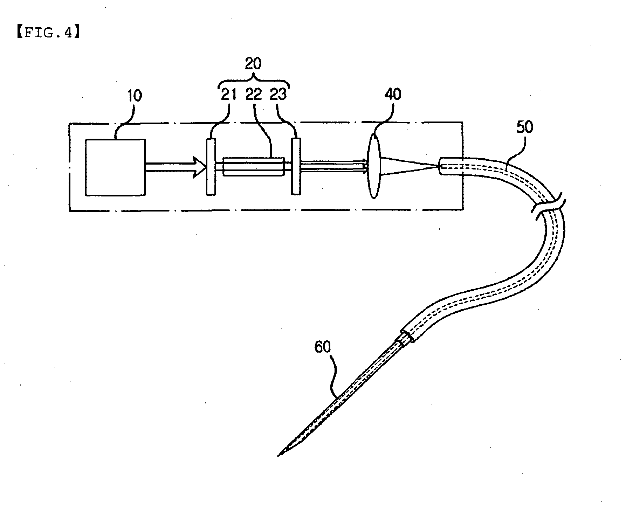

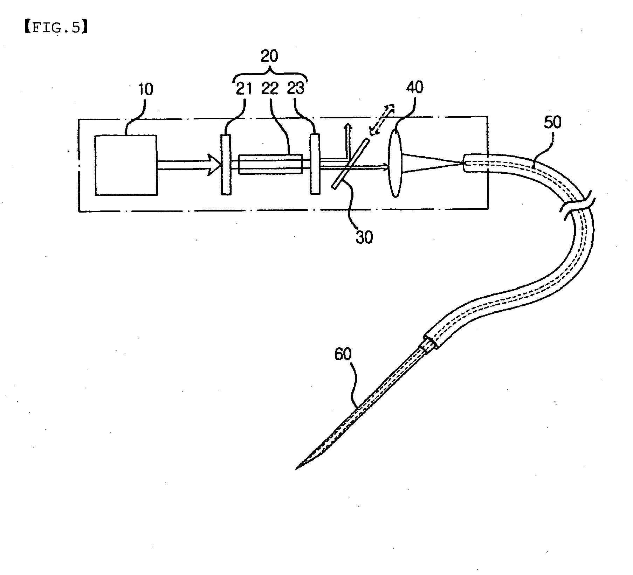

FIG. 3 shows the specific configuration of one embodiment using an apparatus for lipid removal using infrared OPO laser according to the present invention; and FIGS. 4 and 5 illustrate the configuration of an apparatus for lipid removal using infrared OPO laser according to one embodiment of the present invention.

Referring to FIGS. 3 to 5, an apparatus for lipid removal using infrared OPO laser according to one embodiment of the present invention may comprise an OPO laser 20, a convergent lens 40, an optical fiber 50, a plastic-metal needle or a metal needle 60, and a temperature control unit 70. In addition, the apparatus may optionally further comprise a dichroic optical filter 30.

The OPO laser 20 functions to output laser light having a wavelengths of about 1,970-1,990 nm and 2,290-2,310 nm using, as a light source, a pump light from a diode-pumped solid-state laser, which outputs light at a wavelength of about 1,064 nm, or a fiber laser, which outputs light at a wavelength of about 1,054-1,074 nm.

The convergent lens 40 functions to converge laser lights from the OPO laser 20.

The optical fiber 50 functions to guide the laser lights, converged by the convergent lens 40, to fat tissue.

The plastic-metal needle 60 comprises a plastic needle into which one end of the optical fiber 50 is inserted. Alternatively, only a metal needle may be used in place of the plastic-metal needle, and the optical fiber may be inserted therein.

The temperature control unit 70 functions to measure and monitor the temperature of the skin in an area into which the plastic-metal needle 60 is inserted and from which fat tissue is removed (fat removal area), in a contact or non-contact manner, and cool the skin or reduce or block the output of the laser when the measured temperature is higher than a preset safe temperature.

The dichroic optical filter 30, which is optionally mounted, is detachably located in a line along which the lights from the OPO laser 20 progress. It functions to reflect light having a wavelength of about 1,970-1,990 nm, and selectively transmits light having a wavelength of about 2,290-2,310 nm, among the lights output from the OPO laser 20.

Each of the above components will be described in further detail.

As illustrated in FIG. 4, the OPO laser 20 may include: an input mirror unit which receives pump light having a wavelength of about 1,064 nm and reflects infrared light generated in the OPO laser 20; a nonlinear crystal 22, which generates and amplifies OPO laser light of a wavelength of about 1,980 nm and OPO laser light of a wavelength of about 2,300 nm by interaction with the pump light received from the input mirror unit 21; and an output mirror unit 23, which outputs a portion of the infrared lights generated from the nonlinear crystal 22 and reflects the remainder.

Due to this configuration, the two laser lights having different wavelengths can be simultaneously output.

When a fiber laser is used as a source of the light having a wavelength of about 1,064 nm, it can more stably output light and can be miniaturized. In this case, the nonlinear crystal 22 is preferably a periodically poled ferroelectric crystal, such as a periodically poled lithium niobate (PPLN; LiNbO₃) and/or a periodically poled MgO-doped stoichiometric lithium tantalate (PPMgSLT; LiTaO₃), which receives pump light of a wavelength of about 1,064 nm and outputs laser light of a wavelengths of about 1,980 nm and 2,300 nm.

Among the lights from the OPO laser 20, as can be seen in FIG. 1, the infrared light of a wavelength of about 1,980 nm has a coefficient of absorption in water that is the highest next to light having a wavelength of 3,100 nm, and the infrared light of a wavelength of about 2,300 nm has the highest coefficient of absorption in fat.

Fat tissue in a living body does not consist only of fat, but also contains a large amount of water. Thus, when fat tissue is irradiated with both the laser light of a wavelength of 1,980 nm and the light of a wavelength of 2,300 nm from the OPO laser 20, it can be destroyed with high energy efficiency in a short time.

Specifically, in a fat area in which a fat layer 903 is thick, fat in the middle portion of the thick fat layer can be safely liquefied in large amounts using both a laser of a wavelength of about 1,980 nm, which has a high coefficient of absorption in water, and a laser of a wavelength of about 2,300 nm, which has a particularly high coefficient of absorption in fat. On the other hand, the lipolysis of fat in the fat layer 903, which is close to both the skin and a deep muscle layer and is relatively thin, may be performed using a dichroic optical filter 30 as shown in FIG. 5, so that fat close to the skin and muscle can be decomposed as much as possible without damaging the skin or muscle, because the dichroic optical filter 30 reflects the light of wavelength of about 1,980 nm and selectively transmits the light of a wavelength of about 2,300 nm only, which preferentially decomposes fat.

As described above, because the two laser lights having different wavelengths have very high coefficients of absorption in fat tissue, the depths of penetration of the laser lights are very short. Thus, these lights have a low risk of damaging other surrounding tissues and can achieve the removal of fat tissue within a short time.

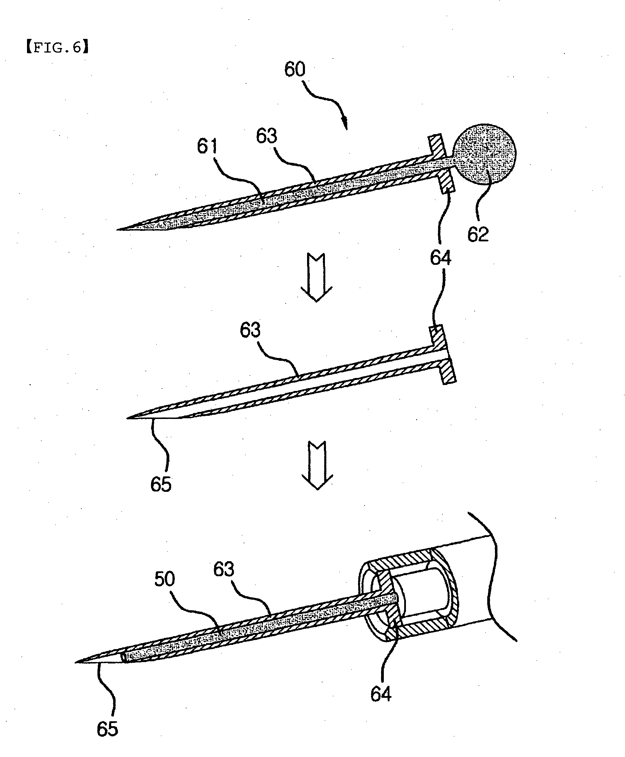

FIG. 6 illustrates the configuration of a plastic-metal needle according to one embodiment of the present invention. Referring to FIG. 6, the plastic-metal needle 60 comprises: a metal needle 61 for skin insertion having a handle 62 formed at one end; and a plastic needle 63, which has a cylindrical structure surrounding the metal needle 61 and in which a protrusion 64 for fixing the optical fiber 50 is formed at one end and a slant 65 is formed at the other end, the plastic needle 63 being inactive *in vivo* and being capable of easily passing a laser.

In this configuration, after the metal needle 61 has been taken out of the plastic-metal needle 60, the laser optical fiber is inserted into the remaining plastic needle such that the fat layer 903 can be irradiated with a laser.

Herein, the end of the optical fiber 50 is inclined such that a laser can be irradiated at various angles, including 30°, 45°, 60°, 90°, 120° and 180°. In addition, the irradiating end of the optical fiber has varying lengths, ranging from about 1 mm to about 40 mm. Thus, the configuration of the optical fiber 50 may vary depending on the output of the laser to be irradiated and the area and thickness of fat to be removed. Accordingly, the appropriate kind of optical fiber may be selected and inserted into the plastic needle in order to remove fat.

Moreover, the metal needle 61 preferably has a diameter of 16-24 G, which leaves no wound even when it pricks the skin.

The use of this plastic-metal needle 60 is as follows: As shown in FIG. 6, when the plastic metal-needle 60 is inserted into the subcutaneous fat layer 903 and the metal needle 61 is then taken out of the plastic-metal needle 60, only the plastic needle 63, through which a laser can easily pass, remains. The slant 65 at the end of the plastic needle 63 is designed to be as long as required, and is caused to face inward in the fat layer 903. Then, the optical fiber is inserted up to the slant 65, and the laser is caused to face inward in the fat layer 903. Then, lasers having wavelengths of about 1,980 nm and about 2,300 nm are oscillated simultaneously or sequentially as required.

After fat has been liquefied as described above, the optical fiber 50 is removed, while only the plastic needle 63 remains. Then, the liquefied fat can be removed by suction through the plastic needle 63.

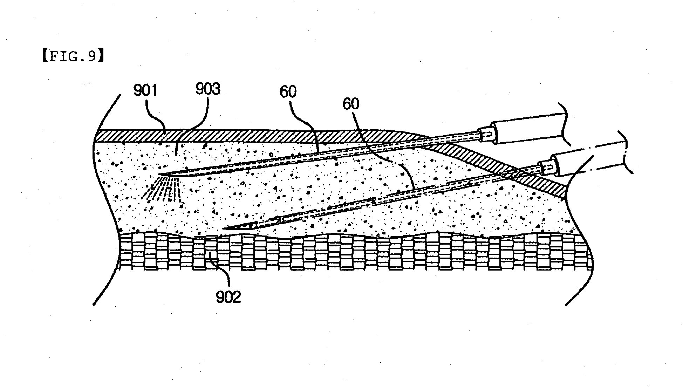

It should be noted that, after the liquefaction of fat in one area has been completed as described above, the same surgery can be repeated in an adjacent area, as shown in FIG. 9. However, it should be noted that, in this operation, the metal needle may be used in place of the plastic needle.

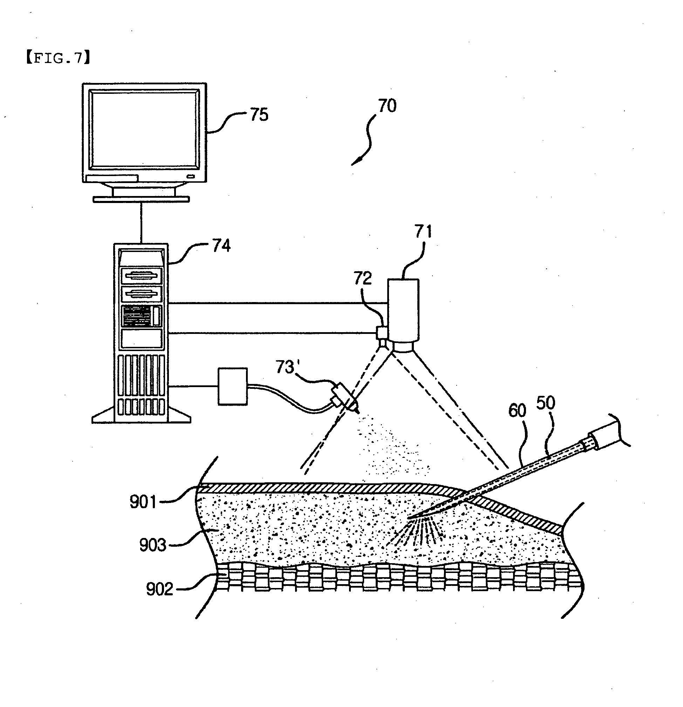

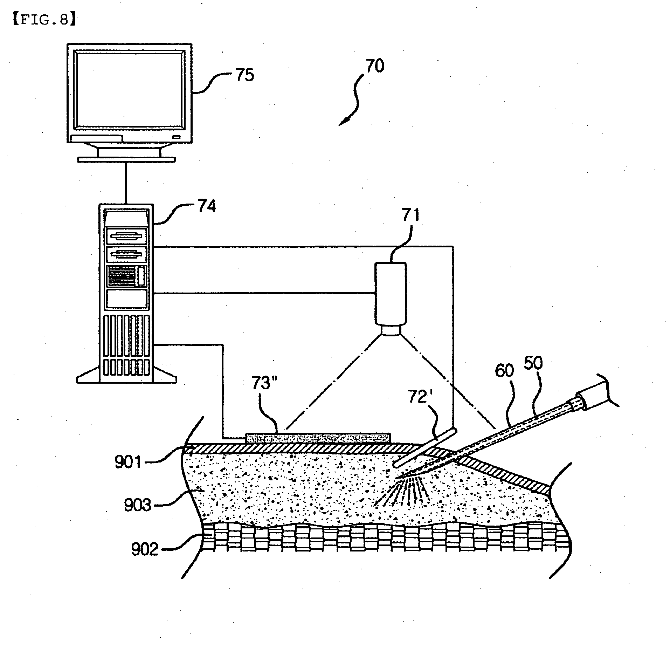

FIGS. 7 and 8 illustrate the configuration of a temperature control unit that is used in the inventive apparatus of removing fat using the OPO laser. Referring to FIGS. 7 and 8, a temperature control unit 70 may comprise a camera 71, a temperature sensor 72, a cooler 73 and a processor 74. In addition, it may further comprise a display 75.

The camera 71 takes an image of the skin in a region from which fat tissue is being removed.

The temperature sensor 72 functions to measure a temperature by receiving light emitted from the skin of the area from which the fat tissue is being removed (fat removal area).

The cooler 73 functions to cool the skin of the fat removal area.

The processor 74 is connected with the camera 71, the temperature sensor 72 and the cooler 73. In the processor 74, a color table for expressing temperature as a color, and a safe temperature, which indicates a skin temperature at which tissues other than the fat to be removed are not damaged, are input. Thus, the processor 74 functions to convert the measured temperature received from the temperature sensor into a color, and to control the cooler 73 so as to reduce the skin temperature of the fat removal area when the measured temperature is higher than the safe temperature.

The display 75 is connected with the processor 74 and functions to display a color corresponding to the measured temperature on the image taken by the camera 71.

Although not shown in FIGS. 7 and 8, it should be noted that the temperature control unit 70 according to one embodiment of the present invention may optionally comprise either a control device which automatically stops when the skin temperature exceeds the safe temperature, which is 44 °C or higher, or a program for this operation, which is programmed in the processor 74.

These components will now be described in further detail. The temperature sensor 72 may be a pyrometer sensor, a radiation sensor or the like, which can measure the surface temperature of the skin in a noncontact manner. It should be noted that a method of measuring the skin temperature in a contact manner using the tip of the temperature sensor 72, inserted into the skin surface and the skin dermal layer or the fat layer 903, may also be used.

The cooler 73 may be composed of either a temperature drop spray, which reduces the surface temperature of the skin according to the control of the processor 74, or a cold air blower, or alternatively, a cooling plate, which is brought into contact with the skin and comprises a Peltier element which is electrically cooled.

As the display 75, any known element may be used without particular limitation, as long as it can display a color corresponding to the measured temperature on the image taken by the camera 71.

In the liquefaction of fat, any change in temperature during the operation is important, and thus it is important to monitor the temperature. Accordingly, the temperature control unit 70 is configured so as to acquire the image and temperature of the skin in a fat removal area using the camera 71 and the temperature sensor 72. The temperature control unit 70 receives the acquired image and temperature, converts the temperature into a color using the color table in the processor 74, displays the color onto the image being input, and displays the image on the display 75. When the input measured temperature is higher than the safe temperature (about 44 °C), the temperature control unit causes the cooler 73 to operate such that the skin temperature is maintained lower than the safe temperature.

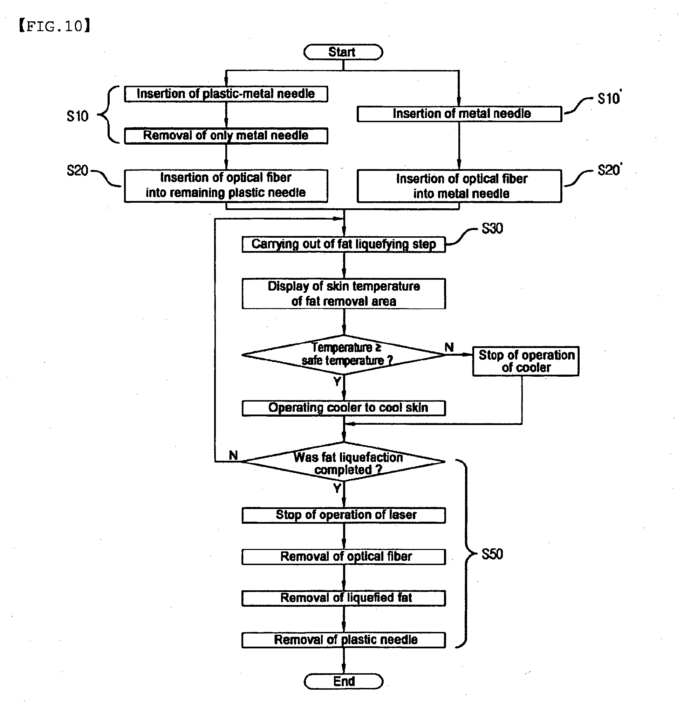

FIGS. 10 to 12 are flow charts illustrating the method of removing fat using the infrared OPO laser. Hereinafter, the method of removing a subcutaneous fat layer using the fat removal apparatus comprising the infrared OPO laser will be described with reference to FIGS. 10 to 12. Further, repeated descriptions will be omitted.

As shown in FIG. 10, the method of removing fat using the infrared OPO laser may comprise an insertion hole-forming step S10, an insertion step S20, and a fat liquefying step S30. It may further comprise a temperature control step S40 and a liquefied-fat removing step S50. Alternatively, it may comprise a metal needle-inserting step S10' and an optical fiber-inserting step S20' in place of steps S10 and S20.

The insertion hole-forming step S10 is a step of inserting the plastic-metal needle 60 into a subcutaneous fat layer while forming an insertion hole in the skin of a patient. The inserting step is a step of removing the metal needle 61 after subcutaneous insertion of the plastic-metal needle 60 and inserting the optical fiber 50 into the remaining plastic needle 63.

Meanwhile, the metal needle-inserting step S 10' is a step of inserting the metal needle into a subcutaneous fat layer while forming an insertion hole in the patient's skin. The optical fiber-inserting step is a step of inserting the optical fiber 50 into the metal needle.

The fat liquefying step S30 is a step of liquefying fat by converging laser light having a wavelength ranging from about 1,054 nm to about 1,074 nm and laser light having a wavelength ranging from about 2,290 nm to about 2,310nm by the convergent lens 40 which receives pump laser light having a wavelength ranging from about 1,054 nm to about 1,074 nm, and delivering the converged laser lights through the optical fiber 50 into the fat layer.

Moreover, the temperature control step S40 is a step of displaying, monitoring and controlling the temperature of the skin in the fat removal region using the temperature control unit 70.

The liquefied-fat removing step S50 is a step of removing residue of the fat, liquefied in the fat liquefying step (30), from the fat removal area by suction through the plastic needle 63.

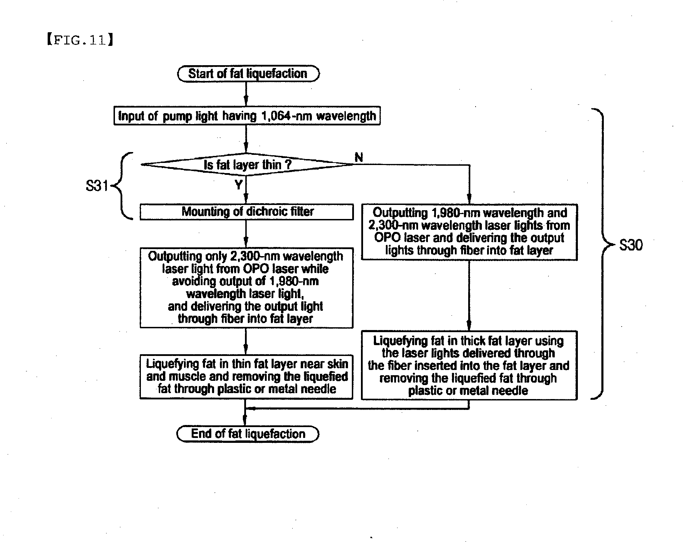

The fat liquefying step S30 will now be described in further detail with reference to FIG. 11. In fat liquefying step S30, in order to remove fat from an area in which the fat layer 903 is thick, pump light having a wavelength of 1,064 nm is input, and laser light having a wavelength of about 1,980 nm and laser light having a wavelength of about 2,300 nm are output through the OPO laser and delivered into the fat layer through the optical fiber. In contrast, in order to remove fat from an area in which the fat layer 903 is thin, the method may further comprise an output light control step S31 of mounting the dichroic optical filter 30 between the OPO laser 20 and the convergent lens 40.

In this case, among the lights output from the OPO laser 20, light having a wavelength ranging from about 1,970 nm to about 1,990 nm is reflected, and light having a wavelength ranging from about 2,290 nm to about 2,310 nm is output and delivered into the fat layer through the optical fiber.

As shown in FIG. 9, the slant 65 of the plastic needle 63 is preferably designed such that light from the OPO laser 20 is directed away from the dermis of the patient during the surgical operation.

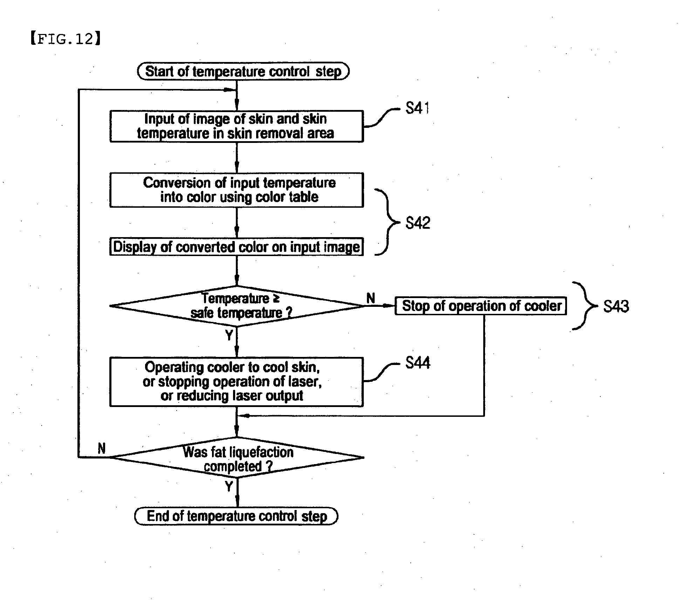

As shown in FIG. 12, the temperature control step S40 may comprise: a measurement step S41 of continuously receiving the image and temperature of an area from which fat is being removed (fat removal area) through the camera 71 and the temperature sensor 72; a conversion step S42 of converting the temperature, input in the measurement step S41, into a color using a color table in the processor 74, and displaying the color on the image; a comparison step S43 of comparing the temperature input in the measurement step S41 with the safe temperature to determine whether the input temperature is higher than the safe temperature; and a control step S44 of operating the cooler 73 to maintain the skin temperature lower than the safe temperature, or stopping laser irradiation, or reducing laser output, when the result of determination in the comparison step S43 indicates that the input temperature is higher than the safe temperature.

Although the preferred embodiments of the present invention have been disclosed for illustrative purposes, those skilled in the art will appreciate that various modifications, additions and substitutions are possible, without departing from the scope of the invention as disclosed in the accompanying claims.

### Industrial Applicability

As described above, the inventive apparatus of removing fat from a living body using the OPO laser can more efficiently and effectively remove fat from a living body, and thus can be applied in various fields, including the medical field and the cosmetic surgery field.

## Claims

1. An apparatus for lipid removal, comprising:
a laser (20) and a pump light source (10) configured to generate a pump light;
an optical fiber (50) configured to guide the light from the laser (20) to fat tissue; and
a needle (60) into which one end of the optical fiber (50) is inserted,
wherein the laser (20) is an OPO laser (20) configured to receive the pump light having a wavelength ranging from 1,054 nm to 1,074 nm and to output a first laser light having a wavelength ranging from 1,970 nm to 1,990 nm and a second laser light having a wavelength ranging from 2,290 nm to 2,310 nm simultaneously,
wherein the apparatus further comprises a dichroic optical filter (30) detachably located in a line along which the light output from the OPO laser (20) progresses and configured to reflect the first laser light and to selectively transmit the second laser light.

2. The apparatus according to claim 1, further comprising:
a convergent lens (40) configured to converge the laser light output from the OPO laser (20);
wherein the optical fiber (50) is configured to guide the laser light converged by the convergent lens (40); and
a temperature control unit (70) configured to display, measure and monitor the temperature of the skin in an area into which the needle (60) is inserted and from which fat is to be removed, and configured to cool the skin in the area or to stop laser irradiation when the measured temperature is higher than a preset safe temperature.

3. The apparatus according to claim 1, further comprising:
a convergent lens (40) configured to converge the light passed through the dichroic optical filter (30) from the OPO laser (20), wherein the optical fiber (50) is configured to guide the laser light converged by the convergent lens (40).

4. The apparatus according to any one of claims 1 to 3, wherein the pump light source is a diode-pumped solid-state laser.

5. The apparatus according to any one of claims 1 to 3, wherein the pump light source is a fiber laser.

6. The apparatus according to any one of claims 1 to 3, wherein the OPO laser (20) comprises:
an input mirror unit (21) configured to receive the pump light and reflect an infrared light generated in the OPO laser (20);
a nonlinear crystal (22) configured to generate and amplify a light generated in the OPO laser (20) by interaction with the pump light received from the input mirror unit (21); and
an output mirror unit (23) configured to output a portion of the infrared light generated by the nonlinear crystal (22) and reflect the remainder.

7. The apparatus according to claim 6, wherein the nonlinear crystal (22) is a periodically poled ferroelectric crystal configured to receive the pump light and output the first laser light and the second laser light.

8. The apparatus according to claim 6, wherein the nonlinear crystal (22) is a periodically poled lithium niobate configured to receive the pump light and output the first laser light and the second laser light.

9. The apparatus according to claim 6, wherein the nonlinear crystal (22) is a periodically poled MgO-doped stoichiometric lithium tantalate configured to receive the pump light and output a first laser light and the second laser light.

10. The apparatus according to claim 1 or 2, wherein the needle (60) comprises:
a metal needle (61) for skin insertion having a handle (62) formed at one end; and
a plastic needle (63) which surrounds the metal needle (61) and has a protrusion for fixing the optical fiber (50) formed at one end and a slant formed at the other end.

11. The apparatus of claim 2, wherein the temperature control unit (70) comprises:
a camera (71) for taking an image of the skin in a fat removal area from which fat tissue is to be removed;
a temperature sensor (72) configured to measure temperature by receiving an infrared light emitted from the skin of the fat removal area;
a cooler (73) for cooling the skin of the fat removal area; and
a processor (74) connected with the camera (71), the temperature sensor (72) and the cooler (73) and in which a color table for expressing the temperature as a color and a safe temperature are input, so that the processor (74) is configured to convert the measured temperature received from the temperature sensor (72) into the color, and control the cooler (73) to reduce the skin temperature of the fat removal area, or stop laser irradiation when the temperature measured is higher than the safe temperature.

12. The apparatus of claim 11, wherein the temperature control unit (70) further comprises a display (75) connected to the processor (74) and configured to display a color corresponding to the temperature measured on the image taken by the camera (71).

## Patentansprüche

1. Vorrichtung zur Fettentfernung, umfassend:
einen Laser (20) und eine Pumplichtquelle (10), die dazu ausgestaltet ist, ein Pumplicht zu erzeugen;
eine optische Faser (50), die dazu ausgestaltet ist, das Licht von dem Laser (20) zu Fettgewebe zu leiten; und
eine Nadel (60), in welche ein Ende der optischen Faser (50) eingeführt ist,
wobei der Laser (20) ein OPO-Laser (20) ist, der dazu ausgestaltet ist, das Pumplicht mit einer Wellenlänge in dem Bereich von 1.054 nm bis 1.074 nm zu empfangen und ein erstes Laserlicht mit einer Wellenlänge in dem Bereich von 1.970 nm bis 1.990 nm und ein zweites Laserlicht mit einer Wellenlänge in dem Bereich von 2.290 nm bis 2.310 nm gleichzeitig auszugeben,
wobei die Vorrichtung des Weiteren einen dichroischen optischen Filter (30) umfasst, der sich abnehmbar in einer Leitung befindet, entlang welcher sich das Licht fortbewegt, das von dem OPO-Laser (20) ausgegeben wird, und dazu ausgestaltet ist, das erste Laserlicht zu reflektieren und das zweite Laserlicht selektiv zu übertragen.

2. Vorrichtung nach Anspruch 1, des Weiteren umfassend:
eine Konvergenzlinse (40), die dazu ausgestaltet ist, das von dem OPO-Laser (20) ausgegebene Laserlicht zu konvergieren;
wobei die optische Faser (50) dazu ausgestaltet ist, das durch die Konvergenzlinse (40) konvergierte Laserlicht zu leiten; und
eine Temperatursteuereinheit (70), die dazu ausgestaltet ist, die Temperatur der Haut in einem Bereich, in welchen die Nadel (60) eingeführt ist, und von welchem Fett entfernt werden soll, anzuzeigen, zu messen und zu überwachen, und dazu ausgestaltet ist, die Haut in dem Bereich zu kühlen oder die Laserbestrahlung anzuhalten, wenn die gemessene Temperatur höher als eine zuvor eingestellte sichere Temperatur ist.

3. Vorrichtung nach Anspruch 1, des Weiteren umfassend:
eine Konvergenzlinse (40), die dazu ausgestaltet ist, das Licht von dem OPO-Laser (20), das durch den dichroischen optischen Filter (30) hindurch geht, zu konvergieren, wobei die optische Faser (50) dazu ausgestaltet ist, das durch die Konvergenzlinse (40) konvergierte Laserlicht zu leiten.

4. Vorrichtung nach irgendeinem der Ansprüche 1 bis 3, wobei die Pumplichtquelle ein diodengepumpter Festkörperlaser ist.

5. Vorrichtung nach irgendeinem der Ansprüche 1 bis 3, wobei die Pumplichtquelle ein Faserlaser ist.

6. Vorrichtung nach irgendeinem der Ansprüche 1 bis 3, wobei der OPO-Laser (20) Folgendes umfasst:
eine Eingangsspiegeleinheit (21), die dazu ausgestaltet ist, das Pumplicht zu empfangen und ein Infrarotlicht zu reflektieren, das in dem OPO-Laser (20) erzeugt wird;
einen nichtlinearen Kristall (22), der dazu ausgestaltet ist, ein Licht zu erzeugen und zu verstärken, das in dem OPO-Laser (20) durch Interaktion mit dem Pumplicht erzeugt wird, das von der Eingangsspiegeleinheit (21) empfangen wird; und
eine Ausgangsspiegeleinheit (23), die dazu ausgestaltet ist, einen Teil des Infrarotlichts auszugeben, das durch den nichtlinearen Kristall (22) erzeugt wird, und den Rest zu reflektieren.

7. Vorrichtung nach Anspruch 6, wobei der nichtlineare Kristall (22) ein periodisch gepolter ferroelektrischer Kristall ist, der dazu ausgestaltet ist, das Pumplicht zu empfangen und das erste Laserlicht und das zweite Laserlicht auszugeben.

8. Vorrichtung nach Anspruch 6, wobei der nichtlineare Kristall (22) ein periodisch gepolter Lithiumniobat ist, der dazu ausgestaltet ist, das Pumplicht zu empfangen und das erste Laserlicht und das zweite Laserlicht auszugeben.

9. Vorrichtung nach Anspruch 6, wobei der nichtlineare Kristall (22) ein periodisch gepolter MgO-dotierter stöchiometrischer Lithiumtantalat ist, der dazu ausgestaltet ist, das Pumplicht zu empfangen und ein erstes Laserlicht und das zweite Laserlicht auszugeben.

10. Vorrichtung nach Anspruch 1 oder 2, wobei die Nadel (60) Folgendes umfasst:
eine Metallnadel (61) zur Einführung in die Haut mit einem Griff (62), der an einem Ende ausgebildet ist; und
eine Kunststoffnadel (63), welche die Metallnadel (61) umgibt und einen Vorsprung zum Befestigen der optischen Faser (50) aufweist, der an einem Ende ausgebildet ist, und eine Schräge aufweist, die an dem anderen Ende ausgebildet ist.

11. Vorrichtung nach Anspruch 2, wobei die Temperatursteuereinheit (70) Folgendes umfasst:
eine Kamera (71) zum Aufnehmen eines Bildes der Haut in einem Fettentfernungsbereich, aus dem Fettgewebe entfernt werden soll;
einen Temperatursensor (72), der dazu ausgestaltet ist, die Temperatur durch Empfangen eines Infrarotlichts zu messen, das von der Haut des Fettentfernungsbereichs ausgestrahlt wird;
eine Kühleinrichtung (73) zum Kühlen der Haut des Fettentfernungsbereichs; und
einen Prozessor (74), der mit der Kamera (71), dem Temperatursensor (72) und der Kühleinrichtung (73) verbunden ist, und in welchen eine Farbtabelle zum Ausdrücken der Temperatur als Farbe und eine sichere Temperatur eingegeben sind, so dass der Prozessor (74) dazu ausgestaltet ist, die gemessene Temperatur, die von dem Temperatursensor (72) empfangen wird, zu der Farbe zu konvertieren, und die Kühleinrichtung (73) zu steuern, um die Hauttemperatur des Fettentfernungsbereichs zu reduzieren oder die Laserbestrahlung anzuhalten, wenn die gemessene Temperatur höher als die sichere Temperatur ist.

12. Vorrichtung nach Anspruch 11, wobei die Temperatursteuereinheit (70) des Weiteren eine Anzeige (75) umfasst, die mit dem Prozessor (74) verbunden und dazu ausgestaltet ist, eine Farbe anzuzeigen, die der Temperatur entspricht, die auf dem Bild gemessen wurde, das mit der Kamera (71) aufgenommen wurde.

## Revendications

1. Appareil permettant une élimination des lipides, comprenant:
- une source laser (20) et une source lumineuse à pompe (10) configurée pour générer une lumière de pompe;
- une fibre optique (50) configurée pour guider la lumière du laser (20) au tissu adipeux; et
- une aiguille (60) dans laquelle une extrémité de la fibre optique (50) est insérée,
- où le laser (20) est un laser d'oscillateur paramétrique optique (20), configuré pour recevoir la lumière de pompe ayant une longueur d'onde allant de 1 054 nm à 1 074 nm et pour faire sortir simultanément une première lumière laser ayant une longueur d'onde allant de 1 970 nm à 1 990 nm et une seconde lumière laser ayant une longueur d'onde allant de 2 290 nm à 2 310 nm,
- où l'appareil comprend de plus un filtre optique dichroïque (30), logé de manière détachable dans une ligne le long de laquelle la sortie de la lumière du laser d'oscillateur paramétrique optique (20) progresse, et configuré pour faire réfléchir la première lumière laser et pour transmettre de manière sélective la seconde lumière laser.

2. Appareil selon la revendication 1, comprenant de plus:
- une lentille convergente (40), configurée pour faire converger la lumière laser sortie du laser d'oscillateur paramétrique optique (20);
- où la fibre optique (50), est configurée pour guider la lumière laser convergée par la lentille convergente (40); et
- une unité de contrôle de la température (70), configurée pour afficher, mesurer et surveiller la température de la peau dans la zone dans laquelle l'aiguille (60) est insérée et de laquelle la graisse doit être éliminée, et configurée pour refroidir la peau dans la zone ou pour faire arrêter l'irradiation à laser quand la température mesurée est plus élevée qu'une température de sécurité définie en préalable.

3. Appareil selon la revendication 1, comprenant de plus:
- une lentille convergente (40), configurée pour faire converger la lumière passée par le filtre optique dichroïque (30) du laser d'oscillateur paramétrique optique (20), où la fibre optique (50) est configurée pour guider la lumière laser convergée par la lentille convergente (40).

4. Appareil selon l'une quelconque des revendications 1 à 3, où la source de lumière à pompe est un laser solide pompé par diode.

5. Appareil selon l'une quelconque des revendications 1 à 3, où la source de lumière à pompe est un laser de fibres.

6. Appareil selon l'une quelconque des revendications 1 à 3, où le laser d'oscillateur paramétrique optique (20) comprend:
- une unité d'entrée à miroir configurée pour recevoir la lumière de pompe et réfléchir une lumière infrarouge générée dans le laser d'oscillateur paramétrique optique (20);
- un cristal non linéaire (22), configuré pour générer et amplifier une lumière générée dans le laser d'oscillateur paramétrique optique (20) par l'interaction avec la lumière de pompe reçue de l'unité d'entrée à miroir (21); et
- une unité de sortie à miroir (23), configurée pour faire sortir une partie de la lumière infrarouge générée par le cristal non linéaire (22) et réfléchir le reste.

7. Appareil selon la revendication 6, où le cristal non linéaire (22) est un cristal ferroélectrique polarisé périodiquement, configuré pour recevoir la lumière de pompe et faire sortir la première lumière laser et la seconde lumière laser.

8. Appareil selon la revendication 6, où le cristal non linéaire (22) est un niobate de lithium polarisé périodiquement configuré pour recevoir la lumière de pompe et faire sortir la première lumière laser et la seconde lumière laser.

9. Appareil selon la revendication 6, où le cristal non linéaire (22) est un tantalate de lithium stoïchiométrique dopé à MgO polarisé périodiquement, configuré pour recevoir la lumière de pompe et faire sortir une première lumière laser et la seconde lumière laser.

10. Appareil selon la revendication 1 ou 2, où l'aiguille (60) comprend:
- une aiguille de métal (61) pour l'insertion de la peau ayant une poignée (62) formée à une extrémité; et
- une aiguille en plastique (63) qui environne l'aiguille de métal (61) et présente une saillie pour fixer la fibre optique (50) formée à une extrémité et une inclinaison formée à l'autre extrémité.

11. Appareil selon la revendication 2, où l'unité de contrôle de la température (70) comprend:
- une caméra (71) pour prendre une image de la peau dans une zone d'élimination de graisse à partir de laquelle le tissu adipeux est pour être éliminé;
- un capteur de température (72), configuré pour mesurer la température en recevant une lumière infrarouge émise de la peau de la zone d'élimination de graisse;
- un refroidisseur (73) pour refroidir la peau de la zone d'élimination de graisse; et
- un processeur (74), connecté avec la caméra (71), le capteur de température (72) et le refroidisseur (73) et où un tableau de couleurs pour exprimer la température comme une couleur et une température de sécurité sont introduits, de sorte que le processeur (74) soit configuré pour convertir la température mesurée reçue du capteur de température (72) dans la couleur, et commander le refroidisseur (73) pour réduire la température de la peau de la zone d'élimination de graisse, ou arrêter l'irradiation à laser quand la température mesurée est plus haute que la température de sécurité.

12. Appareil selon la revendication 11, où l'unité de contrôle de la température (70) comprend de plus un affichage (75), connecté au processeur (74) et configuré pour afficher une couleur correspondant à la température mesurée sur l'image prise par la caméra (71).
